# EUROPÄISCHE PATENTSCHRIFT

(11) **EP 2 217 878 B1**
(45) Veröffentlichungstag und Bekanntmachung des Hinweises auf die Patenterteilung: **27.06.2018**
(21) Anmeldenummer: 08856741.7
(22) Anmeldetag: 04.12.2008
(51) Int. Cl.: G01B 11/25, A61B 5/107, A61B 5/00

(54) **AUFNAHMEVERFAHREN FÜR DAS BILD EINES AUFNAHMEOBJEKTS UND AUFNAHMEVORRICHTUNG**
RECORDING METHOD FOR THE IMAGE OF A RECORDED OBJECT AND RECORDING DEVICE
PROCÉDÉ D'ENREGISTREMENT D'UNE IMAGE D'UN OBJET À ENREGISTRER ET DISPOSITIF D'ENREGISTREMENT

(30) Priorität: 04.12.2007 DE 102007058590
(43) Veröffentlichungstag der Anmeldung: 18.08.2010
(73) Patentinhaber: Sirona Dental Systems GmbH, 64625 Bensheim (DE)
(72) Erfinder: THIEL, Frank, 64372 Ober Ramstadt (DE); FORNOFF, Peter, 64385 Reichelsheim (DE); PFEIFFER, Joachim, 64625 Bensheim (DE)
(74) Vertreter: Sommer, Peter
(86) Internationale Anmeldenummer: PCT/EP2008/066759
(87) Internationale Veröffentlichungsnummer: WO 2009/071611

(56) Entgegenhaltungen:
- WO-A-2005/027770
- WO-A-2007/059780
- DE-A1- 2 410 947
- DE-A1- 10 358 544
- JP-A- 2002 090 126
- US-A- 5 986 745
- US-A1- 2004 151 369

## Beschreibung

### Technisches Gebiet

Die Erfindung betrifft ein Verfahren zur Erstellung eines Bildes eines Aufnahmeobjekts, insbesondere für zahnmedizinische Zwecke, mit den Verfahrensschritten: Projizieren eines Streifenmusters auf das Aufnahmeobjekt, Aufnehmen des projizierten Streifenmusters als Rohbild mit einer Kamera und Berechnen eines Bildes des Aufnahmeobjekts aus dem Rohbild. Das Bild kann dabei insbesondere ein Höhenbild oder ein Intensitätsbild eines präparierten Zahns oder eines Abdrucks davon sein.

Ferner bezieht sich die Erfindung auf eine Vorrichtung zur Durchführung dieses Verfahrens.

### Stand der Technik

Zur direkten optischen dreidimensionalen Vermessung von Objekten, z. B. zur Gewinnung von digitalen Konstruktionsdaten für die rechnergesteuerte Fertigung, kann das Prinzip der Triangulation ausgenutzt werden, bei dem von einer Projektionseinrichtung ein einzelner Lichtstrich oder ein Streifenmuster aus parallelen Strichen auf das aufzunehmende Objekt projiziert und das projizierte Bild unter einem Parallaxenwinkel mit einer Flächenkamera wieder aufgenommen wird. Aufgrund der Oberflächenstruktur der Aufnahmeobjekte erscheint der Streifenverlauf nicht mehr grade, sondern gegenüber den graden Verlauf gekrümmt und verschoben. Aus der Lage der Lichtstrichlinien kann auf die Oberflächenstruktur der Aufnahmeobjekte zurück geschlossen werden.

Zur Erhöhung der Genauigkeit kann die so genannte Phasen-Shift-Triangulationsmethode (phase shifting triangulation) eingesetzt werden. Dabei werden nacheinander mehrere Rohbilder desselben Aufnahmebereichs mit jeweils verschiedenen Positionen der Phasenlage eines Streifengitters erzeugt und aus diesen ein Phasenbild berechnet. Aus dem Phasenbild kann dann mit Hilfe von Kalibrierdaten ein Höhenbild des Aufnahmeobjektes oder auch ein Kontrastbild berechnet werden. Das Höhenbild kann dabei als 3D-Datensatz vorliegen. Um die Bilder exakt berechnen zu können, werden allgemein Streifenmuster mit sinusförmiger Intensitätsverteilung verwendet. Die Triangulation sowie die Phasen-Shift-Triangulationsmethode sind beispielsweise in der US 4,837,732 genauer beschrieben.

Bei der dreidimensionalen Vermessung insbesondere von transluzenten Objekten wie etwa Zähnen mittels Streifenprojektionsverfahren sind die Rohdaten typischerweise von einer schlechteren Qualität als bei der Vermessung von vollständig opaken Objekten. Das heißt unter anderem, dass die an transluzenten Objekten gewonnenen Rohdaten eine niedrigere Signalamplitude besitzen, so dass die daraus gewonnenen dreidimensionalen Daten eine schlechtere Qualität besitzen. Aufgrund des in das Objekt eindringenden und durch diverse Mechanismen rückgestreuten Lichts entsteht bei transluzenten Aufnahmeobjekten ein starkes Untergrundsignal, das hinsichtlich der dreidimensionalen Geometrie der Oberfläche des Objekts keine oder eine verfälschte Information liefert.

Diese störende Wirkung der Transluzenz der Oberfläche auf die in Rohbildern enthaltene dreidimensionale Information über die Oberfläche wird mit größer werdender Gitterperiode des bei der Streifenprojektion verwendeten Gitterstreifenmusters immer geringer. Allerdings hat eine Erhöhung der Gitterkonstanten prinzipbedingt eine Verringerung der Messgenauigkeit zur Folge.

Daher wird heutzutage typischerweise ein Kontrastmittel eingesetzt. Dieses Kontrastmittel ist häufig ein Puder, das auf das zu vermessende Objekt aufgetragen wird und dafür sorgt, dass ein größerer Anteil des eingestrahlten Lichts an der Objektoberfläche reflektiert wird, wodurch sich eine höhere Signalamplitude erhalten lässt. Allerdings führt das Aufbringen eines Kontrastmittels zu einem höheren Aufwand und kann außerdem bei ungleichmäßigem Auftrag die Messgenauigkeit verringern. Die störenden Untergrundsignale bestehen bei allen transluzenten Objekten oder allgemeiner bei allen Objekten, bei denen im Inneren eine Rückstreuung stattfindet.

Aus der DE 103 04 111 A1 wird ein Ansatz zur Erhöhung der Genauigkeit der mittels der Phasen-Shift-Triangulationsmethode gewonnenen Daten offenbart, der auf der Aufteilung von Rohbildern in Gruppen beruht, um aus jeder Gruppe ein Phasenbild zu erstellen. Die so gewonnenen Phasenbilder werden gemittelt, um Störungen unterdrücken, die aus der Periodizität der Gitter stammen. Diese Herangehensweise ist aber bei dem Problem der vermehrten Rückstreuung innerhalb eines Aufnahmeobjekts nicht zielführend. WO2005027770 beschreibt ein Verfahren für schnelle 3D-Abbildung eines Objekts für zahnmedizinische Zwecke durch sequentielle Projizierung von Streifenmustern mit gut unterscheidbaren Streifen. DE2410947 beschreibt ein Verfahren zur Prüfung von Testobjekten auf Formtreue durch Projizieren von gut unterscheidbaren Konturlinien, die durch Vielstrahlinterferenz erzeugt sind, und deren Intensitätsverteilung einer Sinusverteilung mit mindetens einem weiteren Oberwellenterm entspricht.

Der Erfindung, wie sie in den Ansprüchen gekennzeichnet ist, liegt die Aufgabe zugrunde, ein Verfahren der genannten Art zur Aufnahme eines Bildes eines Aufnahmeobjekts anzugeben und eine Vorrichtung zur Erstellung eines Bildes eines Aufnahmeobjekts bereit zu stellen, bei dem sich auch bei Verzicht auf Kontrastmittel eine ausreichende Messgenauigkeit erreichen lässt.

### Darstellung der Erfindung

Diese Aufgabe wird erfindungsgemäß durch das Verfahren nach Anspruch 1 und die Aufnahmevorrichtung nach Anspruch 7 gelöst. Vorteilhafte Ausgestaltungen der Erfindung sind Gegenstand der Unteransprüche.
Die Erfindung baut auf dem Stand der Technik dadurch auf, dass zur Erhöhung der Messgenauigkeit ein Streifenmuster auf das Aufnahmeobjekt projiziert wird, dessen Tastverhältnis kleiner 1 ist. Damit lassen sich Rohbilder für die Berechnung von Höhenbildern oder Intensitätsbildern bereitstellen, wobei bereits abhängig vom jeweiligen Verfahren ein Rohbild ausreichend sein.
Unter Tastverhältnis wird im Folgenden das Verhältnis von ausgeleuchteten bzw. hellen Bereichen zu unausgeleuchteten bzw. dunklen Bereichen durch das aufprojizierte Streifenmuster verstanden. Bei einem Tastverhältnis kleiner 1 hat das aufprojizierte Streifenmuster flächenmäßig mehr dunkle als helle Bereiche.
Die Erfindung beruht darauf, dass bei der Verwendung eines Streifenmusters mit einem Tastverhältnis kleiner 1 das ursprüngliche Muster weniger verbreitert bzw. weniger ausgewaschen wird. Bei gleicher Gitterkonstante führt die Verwendung eines Streifenmusters mit einem Tastverhältnis kleiner 1 zu einer größeren Signalamplitude als ein herkömmlich verwendetes sinusförmiges Streifenmuster mit Tastverhältnis gleich 1 oder ein Streifenmuster mit Tastverhältnis größer 1. Störende Untergrundsignale werden so in einem wesentlichen Umfang verringert, insbesondere bei transluzenten Oberflächen.
Je niedriger das Tastverhältnis ist, desto größer ist der feststellbare positive Effekt. Eine Abnahme der auf das Aufnahmeobjekt treffende und von ihm reflektierten Intensität zu sehr kleinen Tastverhältnis hin kann durch eine entsprechende Verlängerung der Aufnahmezeit bei der Bilderstellung ausgeglichen werden.

In einer bevorzugten Ausführungsform des Verfahrens können das Projizieren des Streifenmusters und das Aufnehmen des projizierten Streifenmusters bei gleicher Ausrichtung der Kamera zum Aufnahmeobjekt und bei mehreren verschiedenen Positionen der Phasenlage des Streifenmusters durchgeführt werden und das Bild kann aus den mehreren gegeneinander phasenverschobenen Kamerarohbildern berechnet werden.

Zur Erhöhung der Genauigkeit werden also nacheinander mehrere Rohbilder mit verschiedenen Positionen der Phasenlage des Gitters erzeugt und aus diesen ein Phasenbild berechnet. Aus dem Phasenbild kann dann, gegebenenfalls mit Hilfe von Kalibrierdaten, ein Höhenbild des Aufnahmeobjektes berechnet werden. Aus den Rohbildern kann aber auch ein Kontrastbild berechnet werden.

Besonders geeignet, insbesondere bei der Phasen-Shift-Triangulationsmethode, sind periodische Streifenmuster. In Bezug auf periodische Streifenmuster kann ein Tastverhältnis kleiner 1 u.a. dann erreicht werden, wenn innerhalb einer Periode die Breite des Bereichs, der mit einer Intensität von ≥50% der maximalen Strahlenintensität ausgeleuchtet wird, kleiner ist als die Breite des Bereichs, der mit einer Intensität von <50% der maximalen Strahlungsintensität ausgeleuchtet wird. Der erstgenannte Bereich entspricht dabei einem Lichtstreifen und der zweitgenannte Bereich einem Dunkelstreifen.

Bevorzugt ist bei periodischen Streifenmustern der Bereich innerhalb einer Periode, der mit einer Intensität von <30% der maximalen Strahlungsintensität ausgeleuchtet wird, größer als der Bereich, der mit einer Intensität von 50% oder mehr der maximalen Strahlungsintensität ausgeleuchtet wird.

Besonders bevorzugt im Hinblick auf eine hohe Messgenauigkeit ist der Bereich innerhalb einer Periode, der mit einer Intensität von <20% der maximalen Strahlungsintensität ausgeleuchtet wird größer als der Bereich, der mit einer Intensität von ≥50% der maximalen Strahlungsintensität ausgeleuchtet wird.

Die Intensitätsverteilung der Hell- und Dunkelstreifen des Streifenmusters entspricht einer Sinusverteilung mit mindestens einem weiteren Oberwellenterm. Derartige Verteilungen haben gegenüber beispielsweise einer Rechteckverteilung den Vorteil, dass bei der Anwendung der Phasen-Shift-Triangulationsmethode, bei der nacheinander mehrere Rohbilder mit verschiedenen Position der Phasenlage des Streifenmusters erzeugt werden, die für herkömmliche Sinusverteilungen verwendeten Formeln zur Auswertung der Rohdaten zu Höhen- oder Kontrastbildern angewendet werden können.
Insbesondere im Bezug auf die Anwendung der Phasen-Shift-Triangulationsmethode hat es sich als vorteilhaft herausgestellt, zur Auswertung und Berechnung des Bildes aus jedem Rohbild mindestens einen Messpunkt zu bestimmen, der in allen Rohbildern einer bestimmten Position in der Projektionsebene entspricht, und die dort gemessenen Intensitäten mit der eingestrahlten Intensitätsverteilung im Rahmen einer Ausgleichsrechnung zu vergleichen, um die Phasenlage der Intensitätsverteilung an dem mindestens einen Messpunkt zu bestimmen und daraus auf die Höhe des Aufnahmeobjekts an dieser Position in der Projektionsebene zu schließen. Eine derartige numerische Auswertung der Daten erlaubt insbesondere auch bei Streifenverteilungen, für die sich keine geschlossenen Formeln zur Auswertung finden lassen, eine möglichst genaue Auswertung der Messdaten.
Eine erfindungsgemäße Aufnahmevorrichtung, die zur Durchführung des beschriebenen Verfahrens geeignet ist, enthält eine Projektionseinrichtung zum Projizieren eines Streifenmusters auf das Aufnahmeobjekt, eine Kamera zum Aufnehmen des projizierten Streifenmusters als Rohbild und Mittel zum Berechnen eines Bildes des Aufnahmeobjektes aus dem mindestens einen Rohbild, wobei die Projektionseinrichtung derart ausgebildet ist, dass sie ein Streifenmuster mit einem Tastverhältnis kleiner 1 projiziert.

Dies geschiet durch die Bereitstellung einer Projektionseinrichtung mit einem entsprechenden Projektionsmuster dessen Breite der lichtdurchlässigen Abschnitte soviel geringer ist als die Breite der lichtundurchlässigen Abschnitte, dass in der Projektionsebene am Aufnahmeobjekt die helleren Bereiche schmaler sind als die dunkleren Bereiche.
Vorteilhafterweise kann das auf das Aufnahmeobjekt zu projizierende Streifenmuster in seiner Position der Phasenlage veränderbar sein, um mehrere Rohbilder unterschiedlicher Phasenlage desselben Aufnahmebereichs bereitzustellen.

### Kurzbeschreibung der Zeichnung

Die Erfindung soll nachfolgend anhand eines Ausführungsbeispiels im Zusammenhang mit den Zeichnungen näher erläutert werden. Es sind jeweils nur die für das Verständnis der Erfindung wesentlichen Elemente dargestellt. Dabei zeigen
- Fig. 1a, b: eine schematische Darstellung einer Aufnahmevorrichtung nach dem Prinzip der Phasen-Shift-Triangulation;
- Fig. 2a bis c: Gegenüberstellungen von verschiedenen projizierten Intensitätsverteilungen mit den jeweils gemessenen Intensitätsverteilungen;
- Fig. 3a bis c: ein Streifenmuster mit gaußartiger Intensitätsverteilung und Tastverhältnis kleiner 1;
- Fig. 4a bis c: ein erfindungsgemäßes Streifenmuster mit einer Verteilung, die einer Sinusfunktion mit dem ersten Oberwellenterm entspricht und ein Tastverhältnis kleiner 1 hat;
- Fig. 5: eine Flussdiagramm einer Ausführungsform des erfindungsgemäßen Aufnahmeverfahrens unter Verwendung der Phasen-Shift-Triangulationsmethode.

### Ausführungsbeispiele

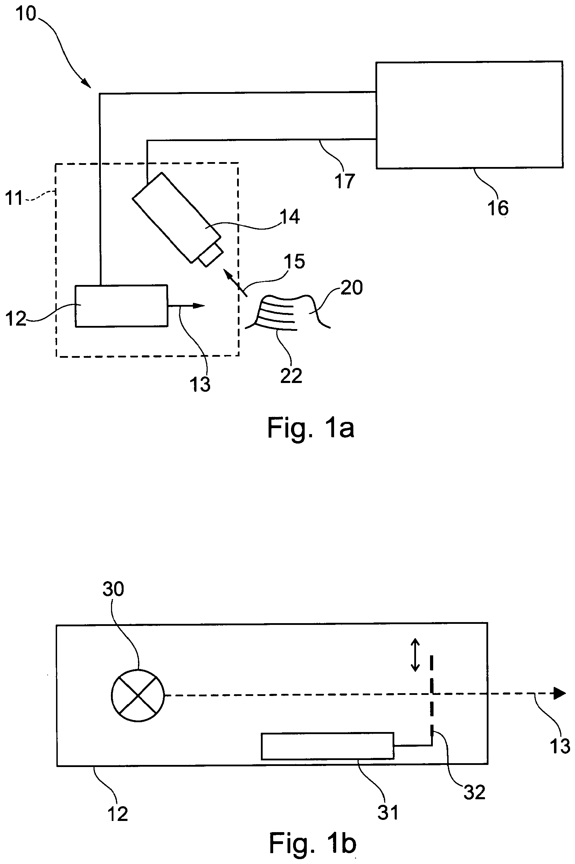

Fig. 1a zeigt in schematischer Darstellung eine allgemein mit 10 bezeichnete Aufnahmevorrichtung, die nach dem Prinzip der Phasen-Shift-Triangulation arbeitet. Die Projektionseinrichtung 12 erzeugt dabei ein Lichtgitter mit parallelen Strichlinien, das auf ein Aufnahmeobjekt, im Ausführungsbeispiel eine Zahnprothese 20, projiziert wird. Aufgrund der dreidimensionalen Oberflächenstruktur des Zahns 20 erscheinen die Lichtgitterlinien auf dem Zahn gekrümmt und unter ungleichmäßigen Abständen. Das über den Projektionsstrahl 13 projizierte Bild 22 wird unter einem Parallaxenwinkel mit einer im Beobachtungsstrahl 15 angeordneten Flächenkamera 14 aufgenommen und zur Auswertung einer Auswerteeinheit 16 zugeführt. Die Projektionseinrichtung 12 und Kamera 14 können auch in einer baulichen Einheit 11 zusammengefasst sein.

Fig. 1b zeigt dabei detaillierter den schematischen Aufbau der Projektionseinrichtung 12. Eine Lichtquelle 30 erzeugt einen Lichtstrahl, der durch ein optisches Gitter 32 durchtritt. Das Gitter 32 ist derart aufgebaut, dass ein Streifenmuster auf die Zahnprothese 20 projiziert wird, bei dem die dunklen Flächen größer als die hellen Flächen sind. Dazu ist das Gitter 32 im vorliegenden Beispiel derart ausgestaltet, dass sich in der Projektionsebene an der Zahnprothese 20 ein Streifenmuster mit der gewünschten Intensitätsverteilung mit Tastverhältnis kleiner 1 ergibt. Der Antrieb 31 lässt sich einsetzen, um im Rahmen der Phasen-Shift-Triangulationsmethode unterschiedliche Phasenlagen des projizierten Streifenmusters einzustellen.

Die aufgenommenen Bilder können über ein Kabel 17 in die Auswerteeinheit 16 ausgelesen und gespeichert werden. Mittels der Auswerteeinheit 16 kann aus mindestens einem Rohbild ein Höhenbild der Zahnprothese 20 berechnet werden, das dann etwa als 3D-Datensatz abgespeichert wird. Die Auswerteeinheit 16 kann dazu Komponenten eines üblichen Computers aufweisen. Nach dem Messvorgang liegt in einem Speicher der mit der Kamera 14 verbundenen Auswerteeinheit 16 ein digitales dreidimensionales Datenmodell der Zahnprothese 20 vor, das als Bild, insbesondere als Kontrastbild dargestellt werden kann oder als Grundlage für eine rechnergesteuerte Konstruktion und Herstellung bzw. Qualitätskontrolle von Zahnersatz dienen kann.

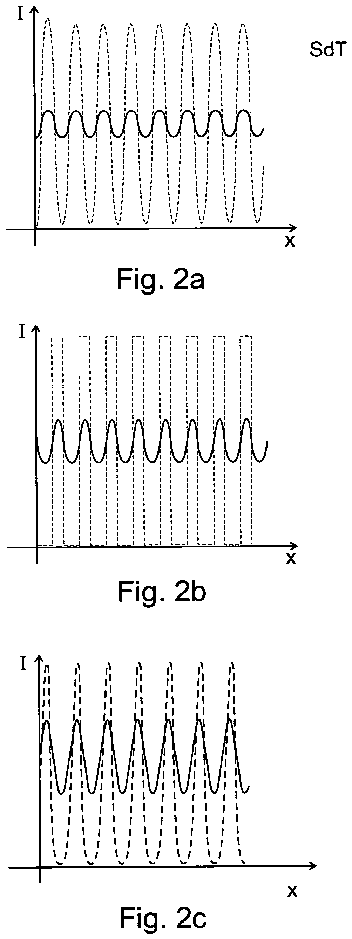

In den Fig. 2a bis c sind verschiedene Intensitätsverteilungen dargestellt, wie sie auf das Aufnahmeobjekt projiziert werden. Dabei handelt es sich in Fig. 2 a um eine herkömmliche sinusförmige Verteilung (gestrichelt), in Fig. 2b um eine Rechteckverteilung (gepunktet) und in Fig. 2c um ein Streifenmuster mit Gaußverteilung (gestrichelt). Alle drei Streifenmuster weisen die gleiche Gitterperiode sowie die gleiche maximale Intensität auf. Sie unterscheiden sich allerdings in ihren Tastverhältnissen. Während bei einer sinusförmigen Verteilung gleich viele Bereiche ausgeleuchtet bzw. hell sind wie unausgeleuchtet bzw. dunkel sind (Fig. 2a, Tastverhältnis = 1), sind bei der Rechteckverteilungen und der Gaußverteilung die Breiten der hellen bzw. ausgeleuchteten Bereiche derart, dass sie kleiner als die unausgeleuchteten bzw. dunklen Bereiche sind (Fig. 2b,c, Tastverhältnis < 1).
Mit einer durchgezogenen Linie sind jeweils die Signale schematisch dargestellt, die nach Reflektion an dem Aufnahmeobjekt unter einem Parallaxenwinkel gemessen wurden. Es zeigt sich, dass bei ansonsten gleichen Bedingungen das gemessene Signal der gemessenen Intensitäten bei einem Tastverhältnis kleiner 1 größer ist als bei einem Tastverhältnis gleich 1, was bei einem Tastverhältnis kleiner 1 zu einer höheren Messgenauigkeit führt. Der Vergleich der Rechteckverteilung mit der Gaußverteilung, die beide die gleiche Halbwertsbreite aufweisen, zeigt, dass durch die weitere Verringerung des intensiv ausgeleuchteten Bereichs zugunsten des dunklen Bereiches bei der Gaußverteilung im Vergleich zur Rechteckverteilung das Tastverhältnis weiter verringert wird und damit eine höhere Signalamplitude bei der gemessenen Intensität erreicht wird.
Während bei einer Rechteckverteilung die typischerweise für eine Sinusverteilung geltenden Formen zur Auswertung der Phasen- bzw. Höheninformation aus dem Signalverlauf nicht anwendbar sind bzw. zu starken systematischen Abweichungen führen, ist dies bei Gittern mit gaußartigen Intensitätsverteilungen nicht der Fall. Bei gaußartigen Intensitätsverteilungen ist der systematische Fehler im Rahmen der notwendigen Messgenauigkeit bei z.B. der Herstellung eines optischen Abdrucks von Zähnen oder anderen Objekten vernachlässigbar.

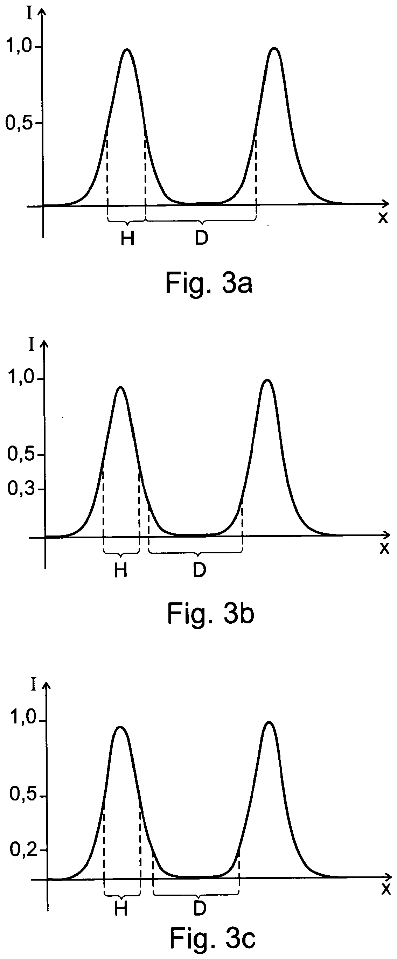

Besonders bei einer gaußartigen Intensitätsverteilung ist ein Tastverhältnis von 1:3, das man erreicht, wenn die Halbwertsbreite ein Viertel der Periode des Streifenmusters beträgt.
Eine periodische gaußartige Intensitätsverteilung mit einem Tastverhältnis von 1:3 ist in den Figuren 3a bis c gezeigt. Zur Illustration des Konzepts des Tastverhältnisses ist eine Möglichkeit dargestellt, das Tastverhältnis quantitativ zu bestimmen. Dazu wird innerhalb einer Periode die Breite H des Bereichs bestimmt, der mit einer Intensität von ≥50% der maximalen Intensität ausgeleuchtet wird. Außerdem wird die Breite D des Bereiches bestimmt, der mit weniger als 50% (Fig. 3a), weniger als 30% (Fig. 3b) bzw. weniger als 20% (Fig. 3c) der maximalen Intensität ausgeleuchtet wird. Um die Messgenauigkeit verglichen mit herkömmlichen Streifenmuster mit Sinusverteilung und Tastverhältnis gleich 1 zu verbessern, sollte der dunkle Bereich immer breiter sein als der helle Bereich. Die Messgenauigkeit lässt sich besonders gut erhöhen, wenn nicht nur der Bereich einer Intensität von <50% breiter als der helle Bereich ist, sondern auch der Bereich mit einer Intensität <30% oder sogar <20% der maximalen Intensität.
Es sei darauf hingewiesen, dass bei sehr kleinen Tastverhältnissen die Belichtungszeit gegebenenfalls verlängert werden muss, um eine hinreichende Gesamtintensität des gemessenen Signals zu erhalten. Besonders kleine Tastverhältnisse werden daher vorzugsweise in Anwendungen eingesetzt, bei denen das Aufnahmeobjekt mit Hilfe einer stationären Aufnahmevorrichtung vermessen wird. In stationäre Aufnahmevorrichtungen können während oder zum Abschluss der Fertigung Zahnprothesen zur Kontrolle vermessen werden.
Bei Anwendungen, bei denen eine bewegliche Aufnahmevorrichtung eingesetzt wird, wie etwa bei der Aufnahme eines Zahns mittels einer Interoralkamera, werden eher etwas höhere Tastverhältnisse bevorzugt, um ein Verwackeln der Rohbilder durch Handzittern beim Halten der Aufnahmevorrichtung zu vermeiden.
An Stelle des in den Fig. 3a bis c dargestellten Streifenmusters mit gaußartiger Intensitätsverteilung ist es auch möglich, andere Gittermuster zu verwenden. Besonders bevorzugt sind Intensitätsverteilungen, bei denen das Streifenmuster im Bereich der von null verschiedenen Intensitätswerte einen eindeutigen Verlauf hat.

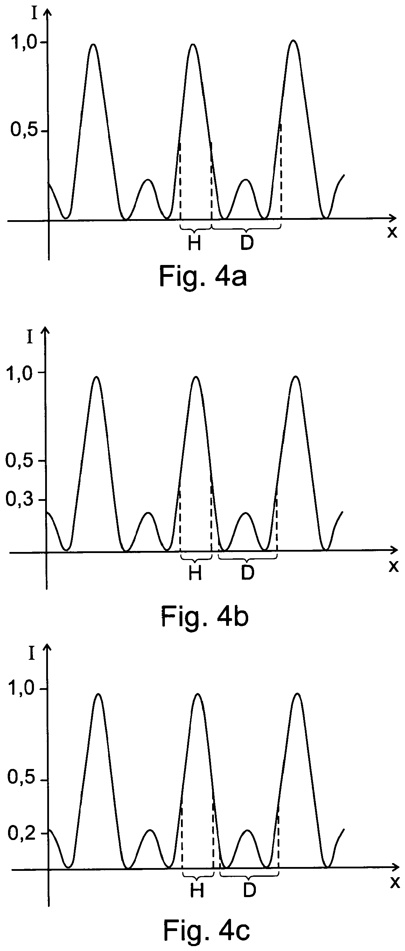

Ein erfindungsgemäßes Streifenmuster ist in den Fig. 4a bis c dargestellt. Es handelt sich dabei um eine Intensitätsverteilung, die einer Sinusverteilung mit dem ersten Oberwellenterm entspricht, d.h. einer Funktion sin(x)+sin(2x). Dieses Streifenmuster ist besonders geeignet, um im Rahmen der Phasen-Shift-Triangulationsmethode verwendet zu werden. Für diese Intensitätsverteilung lässt sich die Phasen- bzw. dreidimensionale Information mithilfe der pixelweisen Auswertung der aus der Literatur bekannten allgemeinen Tangensformel exakt berechnen. Wie in den Fig. 3a bis c wurden auch in den Fig. 4a bis c die Breite der Hell- bzw. Dunkelbereiche einer Periode gekennzeichnet. Auch Verteilungen mit anderen oder mehreren Oberwellentermen sind geeignet, um bei transluzenten Aufnahmeobjekten auch ohne Kontrastmittel eine hinreichende Messgenauigkeit zu erreichen.

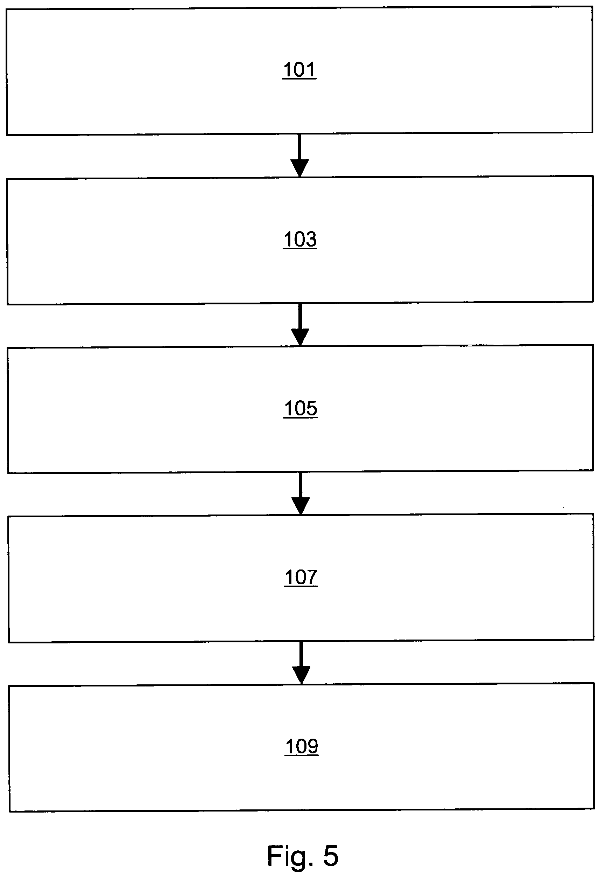

Die Verwendung einer der beschriebenen erfindungsgemäßen Sinusverteilungen des Streifenmusters erhöht die für die Auswertung relevante Signalamplitude in den Rohbildern gerade bei Aufnahmeobjekten mit transluzenten Oberflächen.
Je nach Genauigkeitsanforderung der Anwendung sind verschiedene Auswertungen möglich, mit denen aus dem einen oder mehreren aufgenommenen Rohbildern die Phasen- bzw. dreidimensionale Information generiert werden kann: Man kann eine Auswertung im Sinne einer passiven Triangulation durchführen, bei der ein Rohbild aufgenommen wird. Man kann auch die Phasen-Shift-Triangulationsmethode benutzen und bei geeigneten Intensitätsverteilungen die für Sinusverteilungen bekannten Standardformeln anwenden.
Ebenso kann man bei der Phasen-Shift-Triangulationsmethode eine numerische Herangehensweise für die Auswertung wählen. Eine beispielhafte Ausführungsform ist in Fig. 5 dargestellt. Zunächst werden mindestens zwei Rohbilder eines Aufnahmeobjekts bei unterschiedlicher Phasenlage des Streifenmusters mit Tastverhältnis kleiner 1 in der Projektionsebene am Aufnahmeobjekt mithilfe eines zweidimensionalen Detektors aufgenommen, wobei die Ausrichtung des Detektors bzw, einer Kamera auf das Aufnahmeobjekt bei der Erzeugung der beiden Rohbilder gleich ist (Schritt 101).
Zur Auswertung wird mindestens ein Messpunkt ausgewählt, der in allen Rohbildern einer bestimmten Position in der Projektionsebene entspricht (Schritt 103). Für diesen mindestens einen Messpunkt wird eine Ausgleichsrechnung durchgeführt, bei der die jeweils am Messpunkt in den einzelnen Rohbildern mit der projizierten Intensitätsverteilung verglichen wird (Schritt 105). Aus dem Ergebnis der Ausgleichsrechnung lässt sich die Phasenlage der Intensitätsverteilung an der dem mindestens einen Messpunkt entsprechenden Position in der Projektionsebene ermitteln (Schritt 107). Aus der Phasenlage an der Position in der Projektionsebene der bekannten projizierten Intensitätsverteilung mit Tastverhältnis kleiner 1 lässt sich die Höhe des Aufnahmeobjekts an dieser Position bestimmen (Schritt 109). Dabei wird mit Höhe die Koordinate senkrecht zur Projektionsebene, d.h. im Wesentlichen in Richtung des Projektionsstrahls bezeichnet. Sie ist ein Maß für den Abstand der Oberfläche des Aufnahmeobjekts von der Projektionsebene.

## Patentansprüche

1. Verfahren zur Erstellung eines Bildes eines Aufnahmeobjekts, insbesondere für zahnmedizinische Zwecke, mit den Verfahrensschritten:
a) Projizieren eines Streifenmusters auf das Aufnahmeobjekt, dessen flächenmäßiges Tastverhältnis kleiner 1 ist,
b) Aufnehmen des auf das Aufnahmeobjekt projizierten Streifenmusters als Rohbild mit einer Kamera und
c) Berechnen eines Bildes des Aufnahmeobjekts aus dem Rohbild,
**dadurch gekennzeichnet, dass** auf das Aufnahmeobjekt ein Streifenmuster projiziert wird, dessen Intensitätsverteilung einer Sinusverteilung mit mindestens einem weiteren Oberwellenterm entspricht, wobei das Streifenmuster mittels eines Projektionsmusters erzeugt wird.

2. Verfahren nach Anspruch 1, **dadurch gekennzeichnet, dass** die Schritte a) und b) bei gleicher Ausrichtung der Kamera zum Aufnahmeobjekt und bei mehreren verschiedenen Positionen der Phasenlage des Streifenmusters durchgeführt werden und dass das Bild aus den mehreren gegeneinander phasenverschobenen Rohbildern berechnet wird.

3. Verfahren nach Anspruch 1 oder 2, **dadurch gekennzeichnet, dass** ein periodisches Streifenmuster projiziert wird, bei dem innerhalb einer Periode die Breite des Bereichs, der mit einer Intensität von ≥50% der maximalen Strahlenintensität ausgeleuchtet wird, kleiner ist als die Breite des Bereichs, der mit einer Intensität von <50% der maximalen Strahlungsintensität ausgeleuchtet wird.

4. Verfahren nach Anspruch 3, **dadurch gekennzeichnet, dass** ein periodisches Streifenmuster projiziert wird, bei dem innerhalb einer Periode die Breite des Bereichs, der mit einer Intensität von ≥50% der maximalen Strahlungsintensität ausgeleuchtet wird, kleiner ist als die Breite des Bereichs, der mit einer Intensität von <30% der maximalen Strahlungsintensität ausgeleuchtet wird.

5. Verfahren nach Anspruch 3 oder 4, **dadurch gekennzeichnet, dass** ein periodisches Streitenmuster projiziert wird, bei dem innerhalb einer Periode die Breite des Bereichs, der mit einer Intensität von >50% der maximalen Strahlungsintensität ausgeleuchtet wird, kleiner ist als der Bereich, der mit einer Intensität von <20% der maximalen Strahlungsintensität ausgeleuchtet wird.

6. Verfahren nach einem der Ansprüche 2 bis 5, **dadurch gekennzeichnet, dass** zur Berechnung des Bildes aus jedem Rohbild mindestens ein Messpunkt bestimmt wird, der in allen Rohbildern einer bestimmten Position in der Projektionsebene entspricht, und man die dort gemessenen Intensitäten mit der eingestrahlten Intensitätsverteilung im Rahmen einer Ausgleichsrechnung vergleicht, um die Phasenlage an dem mindestens einen Messpunkt zu bestimmen und daraus auf die Höhe an der Position in der Projektionsebene zu schließen.

7. Aufnahmevorrichtung zur Erstellung eines Bildes eines Aufnahmeobjekts, mit einer Projektionseinrichtung (12) zum Projizieren eines Streifenmusters mit einem flächenmäßigen Tastverhältnis kleiner 1 auf das Aufnahmeobjekt und mit einer Kamera (14) zum Aufnehmen des projizierten Streifenmusters als Rohbild und mit Mitteln (16) zum Berechnen eines Höhenbildes des Aufnahmeobjektes aus mindestens einem Rohbild, **dadurch gekennzeichnet, dass** die Projektionseinrichtung (12) derart ausgebildet ist, dass sie ein Streifenmuster auf das Aufnahmeobjekt projiziert, dessen Intensitätsverteilung einer Sinusverteilung mit mindestens einem weiteren Oberwellenterm entspricht, wobei die Projektionseinrichtung (12) zur Erzeugung des Streifenmusters ein Projektionsmuster aufweist.

8. Aufnahmevorrichtung nach Anspruch 7, **dadurch gekennzeichnet, dass** das auf das Aufnahmeobjekt zu projizierende Streifenmuster in seiner Position der Phasenlage veränderbar ist.

## Claims

1. Method for producing an image of an object to be recorded, in particular for dentistry purposes, comprising the following method steps:
a) projecting a stripe pattern onto the object to be recorded, the areal duty cycle of which is less than 1,
b) recording the stripe pattern projected onto the object to be recorded with a camera as a raw image and
c) calculating an image of the object to be recorded from the raw image,
**characterized in that** a stripe pattern is projected onto the object to be recorded, the intensity distribution of which corresponds to a sinusoidal distribution with at least one other harmonic term, wherein the stripe pattern is created by means of a projection pattern.

2. Method according to Claim 1, **characterized in that** steps a) and b) are carried out with the same alignment of the camera to the object to be recorded and with a plurality of different positions of the phasing of the stripe pattern, and that the image is calculated from the plurality of raw images, which are phase-shifted relative to one another.

3. Method according to Claim 1 or 2, **characterized in that** a periodic stripe pattern is projected, in which, within one period, the width of the region illuminated with an intensity of ≥ 50% of the maximum radiation intensity is smaller than the width of the region illuminated with an intensity of < 50% of the maximum radiation intensity.

4. Method according to Claim 3, **characterized in that** a periodic stripe pattern is projected, in which, within one period, the width of the region illuminated with an intensity of ≥ 50% of the maximum radiation intensity is smaller than the width of the region illuminated with an intensity of < 30% of the maximum radiation intensity.

5. Method according to Claim 3 or 4, **characterized in that** a periodic stripe pattern is projected, in which, within one period, the width of the region illuminated with an intensity of > 50% of the maximum radiation intensity is smaller than the region illuminated with an intensity of < 20% of the maximum radiation intensity.

6. Method according to any of Claims 2 to 5, **characterized in that** at least one measurement point that corresponds to a specific position in the projection plane in all the raw images is determined in each raw image to calculate the image, the intensities measured there are compared with the radiated intensity distribution within the framework of a compensation calculation to determine the phasing at the at least one measurement point and, from that, the height at said position in the projection plane is deduced.

7. Recording device for producing an image of an object to be recorded, comprising a projection device (12) for projecting a stripe pattern with an areal duty cycle of less than 1 onto the object to be recorded, a camera (14) for recording the projected stripe pattern as a raw image and means (16) for calculating a height image of the object to be recorded from at least one raw image, **characterized in that** the projection device (12) is configured in such a way that it projects a stripe pattern onto the object to be recorded, the intensity distribution of which corresponds to a sinusoidal distribution with at least one other harmonic term, wherein the projection device (12) comprises a projection pattern for creating the stripe pattern.

8. Recording device according to Claim 7, **characterized in that** the position of the phasing of the stripe pattern to be projected onto the object to be recorded can be changed.

## Revendications

1. Procédé de création d'une image d'un objet à mettre en image, en particulier à des fins de médecine dentaire, comprenant les étapes de procédé suivantes :
a) la projection, sur l'objet à mettre en image, d'un motif à bandes dont le rapport plein-vide de surface est inférieur à 1,
b) la prise de vue du motif à bandes projeté sur l'objet à mettre en image, sous la forme d'une image brute, à l'aide d'une caméra, et
c) le calcul d'une image de l'objet à mettre en image à partir de l'image brute,
**caractérisé en ce qu'**il est projeté sur l'objet à mettre en image un motif à bandes dont la distribution d'intensité correspond à une distribution sinusoïdale comportant au moins un autre terme d'harmonique, le motif à bandes étant produit au moyen d'un motif de projection.

2. Procédé selon la revendication 1, **caractérisé en ce que** les étapes a) et b) sont réalisées avec une orientation identique de la caméra par rapport à l'objet à mettre en image et à plusieurs positions différentes de la relation de phase du motif à bandes, et **en ce que** l'image est calculée à partir des plusieurs images brutes dont la phase est décalée les unes par rapport aux autres.

3. Procédé selon la revendication 1 ou 2, **caractérisé en ce qu'**il est projeté un motif à bandes périodique dans lequel, pendant une période, la largeur de la zone qui est éclairée avec une intensité ≥ 50 % de l'intensité de rayonnement maximum est inférieure à la largeur de la zone qui est éclairée avec une intensité < 50 % de l'intensité de rayonnement maximum.

4. Procédé selon la revendication 3, **caractérisé en ce qu'**il est projeté un motif à bandes périodique dans lequel, pendant une période, la largeur de la zone qui est éclairée avec une intensité ≥ 50 % de l'intensité de rayonnement maximum est inférieure à la largeur de la zone qui est éclairée avec une intensité < 30 % de l'intensité de rayonnement maximum.

5. Procédé selon la revendication 3 ou 4, **caractérisé en ce qu'**il est projeté un motif à bandes périodique dans lequel, pendant une période, la largeur de la zone qui est éclairée avec une intensité > 50 % de l'intensité de rayonnement maximum est inférieure à la zone qui est éclairée avec une intensité < 20 % de l'intensité de rayonnement maximum.

6. Procédé selon l'une des revendications 2 à 5, **caractérisé en ce que**, pour le calcul de l'image à partir de chaque image brute, il est déterminé au moins un point de mesure correspondant, dans toutes les images brutes, à une position précise dans le plan de projection, et les intensités mesurées à cet endroit sont comparées à la distribution d'intensité rayonnée dans le cadre d'un calcul de compensation, afin de déterminer la relation de phase au niveau de l'au moins un point de mesure et pour en conclure la hauteur au niveau de la position dans le plan de projection.

7. Dispositif de mise en image pour la création d'une image d'un objet à mettre en image, comprenant un système de projection (12) destiné à projeter un motif à bandes avec un rapport plein-vide de surface inférieur à 1 sur l'objet à mettre en image et comprenant une caméra (14) pour la prise de vue du motif à bandes projeté, sous la forme d'une image brute, et comprenant des moyens (16) pour le calcul d'une image de hauteur de l'objet à mettre en image à partir d'au moins une image brute, **caractérisé en ce que** le système de projection (12) est conçu de manière à projeter, sur l'objet à mettre en image, un motif à bandes dont la distribution d'intensité correspond à une distribution sinusoïdale comportant au moins un autre terme d'harmonique, le système de projection (12) présentant un motif de projection pour la création du motif à bandes.

8. Dispositif de mise en image selon la revendication 7, **caractérisé en ce que** le motif à bandes à projeter sur l'objet à mettre en image est modifiable dans sa position de relation de phase.
